(19) Europäisches Patentamt
European Patent Office
Office européen des brevets

(11) Numéro de publication: **0 301 975**
**A2**

(12) # DEMANDE DE BREVET EUROPEEN

(21) Numéro de dépôt: **88401983.7**

(22) Date de dépôt: **29.07.88**

(51) Int. Cl.4: **A 61 K 31/35**

(30) Priorité: **30.07.87 FR 8710800**

(43) Date de publication de la demande:
**01.02.89 Bulletin 89/05**

(84) Etats contractants désignés:
**AT BE CH DE ES FR GB GR IT LI LU NL SE**

(71) Demandeur: **Bonnaure-Mallet, Martine Andrée**
**31, rue Jean Guéhénno**
**F-35700 Rennes (FR)**

(72) Inventeur: **Bonnaure-Mallet, Martine Andrée**
**31, rue Jean Guéhénno**
**F-35700 Rennes (FR)**

(74) Mandataire: **Schrimpf, Robert et al**
**Cabinet Regimbeau 26, Avenue Kléber**
**F-75116 Paris (FR)**

Le titre de l'invention a été modifié (Directives relatives à l'examen pratiqué à l'OEB, A-III, 7.3)

(54) **Monomère et/ou oligomère procyanidolique pour le traitement de maladies parodontales.**

(57) La présente invention concerne une composition destinée au traitement et à la prévention des maladies parodontales caractérisée en ce qu'elle comprend, à titre de principe actif, au moins un monomère et/ou un oligomère procyanidolique.

EP 0 301 975 A2

## Description

# COMPOSITION DESTINEE AU TRAITEMENT ET A LA PREVENTION DES MALADIES PARODONTALES COMPORTANT A TITRE DE PRINCIPE ACTIF, AU MOINS UN MONOMERE ET/OU OLIGOMERE PROCYANIDOLIQUE

La présente invention concerne des compositions destinées au traitement et à la prévention des maladies parodontales qui comportent, à titre de principe actif, au moins un monomère et/ou oligomère procyanidolique.

La parodonte est l'ensemble des tissus de soutien de la dent. Au niveau de la jonction dent-gencive, il existe un espace, appelé sulcus gingivo-dentaire qui est fermé par l'attache épithélio-conjonctive, laquelle constitue une barrière entre le milieu extérieur et le milieu interne.

La poche parondontale, conséquence de la parodontite se traduit par un accroissement anormal de la profondeur du sulcus gingivo-dentaire, dû à des réactions inflammatoires successives qui provoquent la dégénérescence des tissus conjonctifs.

L'absence d'hygiène bucco-dentaire conduit à une accumulation de la plaque à la jonction dent-gencive et dans les espaces interdentaires. L'augmentation en volume de la plaque provoque une gingivite qui évolue le plus souvent vers la parodontite.

Le traitement de la parodontite consiste à éliminer les irritants locaux (plaque dentaire sous-gingivale) et à restaurer l'architecture osseuse et gingivale. Les moyens utilisés jusqu'ici consistent essentiellement à irriguer quotidiennement les poches parodontales avec une solution de principe actif anti-bactérien ou à introduire dans les poches des capsules contenant un principe actif libéré progressivement.

La présente invention a pour but de proposer une nouvelle composition utile dans le traitement et la prévention des maladies parodontales. Cette composition comporte, à titre de principe actif, au moins un monomère et/ou un oligomère procyanidolique qui sera appelé ci-après OPC par abréviation. L'oligomère procyanidolique peut-être d'origine naturelle.

Par l'expression "procyanidolique", on entend les produits de condensation qui comportent la structure flavane 3-ol. Cette définition est valable pour l'ensemble de la description qui va suivre et des revendications.

La (+) catéchine ou 3, 3', 4', 5, 7- flavenepentol de formule :

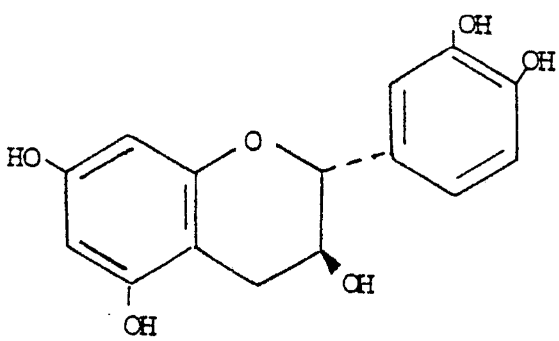

appartient à cette classe de produits.

Dans le cadre de la présente invention, la (+) catéchine est un principe actif particulièrement préféré.

Les compositions, objet de la présente invention preuvent se présenter sous de nombreuses formes utilisables en odontologie notamment sous la forme d'un gel, d'un bain de bouche ou d'une solution injectable intra-gingivale, ou encore d'une solution d'irrigation intra-sulculaire.

Les compositions selon l'invention peuvent être préparées en utilisant des excipients connus dans l'état de la technique et compatibles avec les oligomères procyanidoliques.

Ces compositions selon l'invention comportent de préférence jusqu'à 2 % en poids de principe actif et de préférence environ 1 % en poids. En dessous de 0,05 % en poids, l'activité de la composition peut devenir insuffisante.

L'invention concerne également l'utilisation d'un monomère et/ou d'un oligomère procyanidolique pour la préparation d'une composition destinée aux traitements et à la prévention des maladies parodontales.

Enfin, l'invention concerne un procédé de préparation de composition destinée aux traitement et à la prévention des maladies parondontales caractérisé en ce qu'on mélange un monomère et/ou un oligomère procyanidolique avec un support acceptable en odontologie.

L'exemple ci-dessous illustre la présente invention et montre l'intérêt de l'emploi des 0CP dans le traitement d'une parodontite.

EXEMPLE

1) Création d'un modèle inflammatoire chez le rat

Les expérimentations détaillées ci-dessous ont été réalisées sur des rats albinos de souche Wistar-Commentry, vivant dans des locaux maintenus à température et à degré hygrométriques constants.

1.a) Definition des lots

Les animaux d'un poids moyen de 200 grammes, âgés de 6 semaines en début d'expérimentation, ont été répartis en quatre groupes.
. Dans le lot 1, les animaux subissent une impaction entre la première et la deuxième molaire droites au maxillaire supérieur. L'impaction est réalisée par mise en place d'un coin de bois au niveau du point de contact ; le coin le plus aigu est enfoncé dans l'espace interdentaire jusqu'à blanchiment de la gencive adjacente. Dans certains cas, la pression exercée engendre un léger saignement. Cette intervention est faite sous loupe binoculaire à l'aide d'une précelle et de pinces à écarter les joues ; les rats sont endormis à l'éther.
. Dans le lot 2, les animaux sont seulement soumis à un régime hyperglucidique 2000 de KEYER (UAR, France). Cet aliment est présenté sous forme pulvérulente ; il a été transformé en biscuit par adjonction d'eau.
La composition du régime hyperglucidique est la suivante (pourcentage en poids) :
Saccharose 56 %
Lait en poudre écrémé 28 %
Farine complète de blé 6 %
Levure de bière 4 %
Luzerne en poudre 3 %
Poudre de foie 1 %
Chlorure de sodium 2 %
. Dans le lot 3, les animaux subissent à la fois l'impaction et le régime hyperglucidique.
. Le lot 4 est un groupe de témoins sans aucun traitement.

1.b) Evolution et modification élastiques lors de l'inflammation.
. Lot 1 : L'impaction a entrainé une gêne à l'alimentation, dont témoigne la perte de poids des animaux. La gencive palatine ne montre aucun signe d'inflammation. Après deux semaines, certains animaux ont perdu le matériel d'impaction. La cicatrisation comble progressivement la brèche. Au bout de cinq qemaines, les animaux sont identiques aux animaux témoins (lot 4).
. Lot 2 : Les animaux de ce groupe sont nourris au régime KEYES 2000. L'utilisation de ce régime est une des méthodes les plus fréquemment employées pour induire une maladie parodontale. La plaque bactérienne recouvre progressivement toutes les surfaces dentaires.Le chorion est, dans sa partie superficielle, infiltré de cellules inflammatoires.
. Lot 3 : Ces animaux sont nourris au régime de KEYES 2000 et ont subi, en plus, une impaction. Le poids corporel baisse et l'état général se dégrade, avec un début de chute de poils. Au bout de deux semaines, la totalité des molaires est recouverte de plaque bactérienne. Les altérations gingivales sont visibles. Après cinq semaines, on repère un oedème considérable avec une gencive dehiscente.
Le modèle d'inflammation décrit en 1.a) montre :

une réponse inflammatoire fugace lors d'une impaction induite ; les animaux cicatrisent rapidement ; le réseau élastique lésé se reconstitue de la profondeur vers la surface ;

l'association du régime de KEYES 2000 et d'une effraction gingivale perturbe rapidement l'organisation du parodonte.

2) Injection d'une solution d'oligomères procyanidoliques (OPC)

Dix microlitres d'une solution d'OPC (dimère de catéchine) à 1% en poids dans du sérum physiologique sont injectés dans la papille interdentaire du côté palatin tous les trois jours. Cette répétition de l'injection permet de suivre l'évolution de la maladie parodontale.
Les sept animaux utilisés ont survécu tout au long de l'expérimentation. Leur activité est identique à celle des animaux témoins. Leur poids est resté constant et leur pelage normal.

3) Résultats

3.a) A l'examen endobuccal

A 3 semaines, l'inflammation dans le territoire impacté est discrète, à peine perceptible sous loupe binoculaire. Il se produit un début de rejet du coin de bois. A 5 semaines, on note chez deux animaux, une différence de l'aspect de la muqueuse palatine du côté injecté par rapport au côté opposé.

3.b) A l'examen histologique

Les colorations topographiques montrent l'absence quasi totale d'inflammation. L'épithélium est normal, la kératinisation est légèrement augmentée. Il n'existe pas de cellules inflammatoires.

Après coloration appropriée, les formations élastiques sont nombreuses. Ce sont des éléments enchevêtrés, de faible longueur qui siègent dans la partie moyenne du prélèvement. Sous l'épithélium, qui borde la brèche créée par le coin du bois, les formations élastiques sont les unes longues, épaisses ; les autres courtes et minces.

3.c) En microscopie électronique en transmission

On retrouve les éléments extracellulaires d'un conjonctif gingival normal. Les fibres élastiques et préélastiques sont très denses aux électrons. La limitante élastique des vaisseaux est très contrastée.

Les formations élastiques sont aisément identifiables entre les trousseaux collagènes, à proximité des fibroblastes de la région moyenne et profonde du chorion. La substance amorphe qui les constitue, tannée par les OPC, est semblable à celle que l'on peut voir chez les témoins sains.

On a déterminé la fraction d'aire $A_A$ dans le chorion gingival.
$A_A$ se définit comme suit :

$$A_A \quad \frac{A_O}{A_T}$$

Avec $A_O$ = aire occupée par les éléments élastiques,
$A_T$ = surface de champ analysée.
Pour les animaux de lot 3, traités avec une solution d'OPC, on trouve $A_A = 11{,}1 \pm 0{,}6$ %. Ce résultat est à comparer aux valeurs $A'_A = 9{,}7 \pm 0{,}5$ %, déterminée pour les animaux témoins sains (lot 4) et $A''_A = 5{,}1 \pm 0{,}3$ % pour les animaux impactés (lot 1).
Ces résultats montrent que l'utilisation d'OPC dans le traitement de la parodontite permet de restaurer rapidement la région buccale lésée. On observe une disparition de l'inflammation par rapport aux sujet témoins impactés et une protection des formations élastiques.

Dans le cadre de la présente invention, la composition peut également être sous la forme d'un bain de bouche, d'un gel ou d'une solution d'irrigation intra-sulculaire pour le traitement non chirurgical des poches parodontales. Par solution d'irrigation intra-sulculaire, on entend une solution injectable au moyen d'une pipette ou tout autre moyen, (hydropropulseur, seringue...) à l'intérieur des poches parodontales ou au niveau du rebord gingival.

**Revendications**

1. Composition destinée au traitement et à la prévention des maladies parodontales caractérisée en ce qu'elle comprend, à titre de principe actif, au moins un monomère et/ou un oligomère procyanidolique.
2. Composition selon la revendication 1, caractérisée en ce que ledit principe actif est la (+) catéchine.
3. Composition selon l'une des revendications 1 et 2, caractérisée en ce qu'elle se présente sous la forme d'un gel.
4. Composition selon l'une des revendications 1 et 2, caractérisée en ce qu'elle se présente sous la forme d'un bain de bouche.
5. Composition selon l'une des revendications 1 et 2, caractérisée en ce qu'elle se présente sous la forme d'une solution injectable, intra-gingivale.
6. Composition selon l'une des revendications 1 et 2, caractérisée en ce qu'elle se présente sous la forme d'une solution d'irrigation intra-sulculaire.
7. Composition selon l'une des revendications 1 à 6, caractérisée en ce qu'elle comporte jusqu'à environ 2 % en poids de principe actif.
8. Composition selon la revendication 7, caractérisée en ce qu'elle comporte environ 1 % en poids de principe actif.